# EUROPEAN PATENT APPLICATION

(11) **EP 4 830 707 A1**
(43) Date of publication of application: **28.10.2026**
(21) Application number: 24907471.7
(22) Date of filing: 18.12.2024
(51) Int. Cl.: A61K 31/4725, A61P 27/02, A61P 37/06

(54) **PHARMACEUTICAL COMPOSITION FOR PREVENTING/TREATING OCULAR COMPLICATION IN CHRONIC GRAFT-VERSUS-HOST DISEASE**

(30) Priority: 19.12.2023 JP 2023213755
(71) Applicant: D. Western Therapeutics Institute, Inc., Nagoya-shi, Aichi 460-0003 (JP); Keio University, Tokyo, 108-8345 (JP)
(72) Inventor: KUNO, Noriyuki, Nagoya-shi, Aichi 460-0003 (JP); SUMI, Kengo, Nagoya-shi, Aichi 460-0003 (JP); OGAWA, Yoko, Tokyo 160-8582 (JP); ASAI, Kazuki, Tokyo 160-8582 (JP); SATO, Shinri, Tokyo 160-8582 (JP); HIRAYAMA, Masatoshi, Tokyo 160-8582 (JP); NEGISHI, Kazuno, Tokyo 160-8582 (JP)
(74) Representative: Wächtershäuser & Hartz Patentanwaltspartnerschaft mbB
(86) International application number: PCT/JP2024/044872
(87) International publication number: WO 2025/135087

(57) **Abstract**

The present invention relates to a pharmaceutical composition for preventing/treating an ocular complication in cGVHD, the composition comprising (R)-6-(2-methyl-1,4-diazocan-1-ylsulfonyl)isoquinoline of the following formula (1): 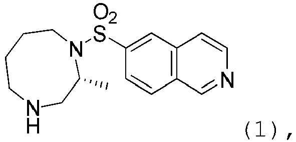 an acid addition salt thereof, or a solvate of the compound or the acid addition salt, as an active ingredient.

## Description

### Technical Field

The present invention relates to a pharmaceutical composition for preventing/treating ocular complications in chronic graft-versus-host disease (cGVHD).

### Background Art

In recent years, hematopoietic stem cell transplantation, such as bone marrow transplantation, is widely used as a treatment for leukemia, severe aplastic anemia, and other blood disorders caused by abnormalities in hematopoietic stem cells. While the success rate of hematopoietic stem cell transplantation has improved, and long-term survival has increased, cGVHD, which is a late complication after transplantation, has become a problem (see Non Patent Literature 1). cGVHD is a systemic immune response triggered against the host by the maturation, in the host, of hematopoietic stem cells transplanted from the donor, and occurs in a wide variety of tissues and organs, including the eyes, mouth, kidneys, lungs, liver, small intestine, and skin. Further, although immunosuppressants are generally administered to post-transplant patients, cGVHD occurs in more than half of the patients; thus, prevention and treatment of cGVHD after hematopoietic stem cell transplantation are extremely important.

cGVHD occurs in various tissues and organs, and symptoms in the eye (ocular complications) include dry eyes, meibomian gland dysfunction, retinal hemorrhage, acute conjunctivitis, and the like, the most frequent of which is dry eyes. Hematopoietic stem cell transplantation is a new treatment, and therefore, the onset mechanism of cGVHD, which is a late complication after transplantation, has not been fully elucidated. As pathological findings of the ocular complications mentioned above, pathological analysis of lacrimal gland tissue shows activation of immunocompetent cells, such as T cells and lymphocytes, and subsequent repair processes, which are observed locally in the lacrimal gland, suggesting that excessive fibrosis of the lacrimal gland due to the interaction of immune networks is the main cause of the pathogenesis; however, there are still many unclear points.

Currently, ocular complications in cGVHD are treated by using eye ointments or oral medications of the immunosuppressant FK506, or steroid eye drops, which are immunomodulators. However, immunosuppressants have many problems with long-term continuous use, and steroid eye drops are difficult to use for a long period of time due to concerns about side effects, such as increased intraocular pressure and cataracts. For these reasons, they are generally not administered after a certain period of time and are replaced by symptomatic treatments. As such symptomatic treatments, hyaluronic acid preparations, vitamin A, steroid preparations, and other eye drops, as well as serum eye drops and artificial tears have been used; however, their effects are not always sufficient, and there is a demand for novel drugs which are highly effective and can be used continuously. Ocular complications in cGVHD occur in about half of the hematopoietic stem cell transplantation patients. Furthermore, once ocular complications occur, they are generally difficult to treat, and about half of the patients who develop ocular complications become severe within a short period of about 3 months (see, for example, Non Patent Literature 2). Therefore, it is important to prevent the onset of ocular complications in cGVHD in treatment after hematopoietic stem cell transplantation, such as bone marrow transplantation, as well as to improve the symptoms and prevent aggravation after the onset. Methods and drugs for these purposes are strongly desired.

Rho kinase (hereinafter referred to as ROCK) is a serine/threonine protein kinase identified as a target protein of the low-molecular-weight GTP-binding protein Rho, and there are two isoforms, ROCK1 and ROCK2, which share high homology of 90% or more in the ATP-binding site. ROCK is involved in various important physiological functions, such as cell contraction, proliferation, migration, and gene expression induction. In recent years, ROCK is attracting attention as a target enzyme for therapeutic drugs against diseases, such as cardiovascular disease, glaucoma, neurodegenerative disease, and cancer, and several ROCK inhibitors have been applied clinically to date (Non Patent Literature 3). Of these, belumosudil is used as a therapeutic drug for cGVHD; however, its therapeutic effects for dry eyes through eye-drop administration are not known. Similarly, ripasudil, which is a ROCK inhibitor, is applied clinically as eye drops for glaucoma treatment; however, its therapeutic effects for the ocular complications mentioned above are not known.

(R)-6-(2-methyl-1,4-diazocan-1-ylsulfonyl)isoquinoline is a compound represented by the following formula (1): 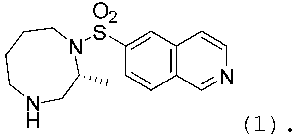 Patent Literature 1 describes that the above compound is effective as the active ingredient of pharmaceuticals for preventing or treating diseases or disorders caused by glaucoma, cardiovascular disease, or neurodegeneration or nerve damage. However, its preventive or therapeutic effects for ocular complications in cGVHD after hematopoietic stem cell transplantation are not known at all.

### Citation List

### Patent Literature

Patent Literature 1: WO 2010/146881

### Non Patent Literature

Non Patent Literature 1: Yoko Ogawa, Ophthalmology, 2003, vol. 45, pp. 309-322
Non Patent Literature 2: Yoko Ogawa, et al., Br. J. Ophthalmol., 1999, vol. 83, pp. 1125-1130
Non Patent Literature 3: J. Med. Chem. 2016, 59, 2269-2300

### Summary of Invention

### Technical Problem

An object of the present invention is to provide a pharmaceutical composition for preventing/treating ocular complications in cGVHD.

### Solution to Problem

The present inventors examined the enzyme inhibitory effects of the compound 1 on ROCK1 and ROCK2. As a result, they have found that the compound 1 inhibits ROCK1 and ROCK2 with IC₅₀ of 0.52 µM and 0.23 µM, respectively. Furthermore, in examining the pharmacological effects based on the ROCK inhibitory effects, the present inventors have found that the compound 1 has the effect of improving ocular complications in cGVHD model mice by eye-drop administration. Thus, the present invention has been completed.

That is, the present invention provides the following inventions [1] to [8].
[1] A pharmaceutical composition for preventing/treating an ocular complication in cGVHD, the composition comprising (R)-6-(2-methyl-1,4-diazocan-1-ylsulfonyl)isoquinoline of the following formula (1): 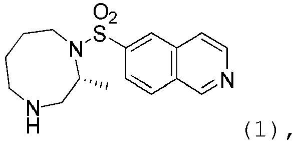 an acid addition salt thereof, or a solvate of the compound or the acid addition salt, as an active ingredient.
[2] The pharmaceutical composition according to [1], wherein the composition is in a form of an eye drop composition.
[3] (R)-6-(2-methyl-1,4-diazocan-1-ylsulfonyl)isoquinoline of the following formula (1): 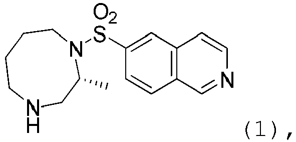 an acid addition salt thereof, or a solvate of the compound or the acid addition salt, for preventing/treating an ocular complication in cGVHD.
[4] The compound according to [3], wherein the pharmaceutical is in a form of an eye drop.
[5] Use of (R)-6-(2-methyl-1,4-diazocan-1-ylsulfonyl)isoquinoline of the following formula (1): 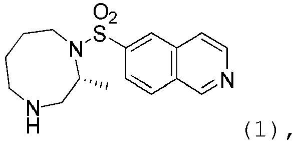 an acid addition salt thereof, or a solvate of the compound or the acid addition salt, for producing a drug for preventing/treating an ocular complication in cGVHD.
[6] The use according to [5], wherein the pharmaceutical is in a form of an eye drop.
[7] A method for preventing/treating an ocular complication in cGVHD, the method comprising administering (R)-6-(2-methyl-1,4-diazocan-1-ylsulfonyl)isoquinoline of the following formula (1): 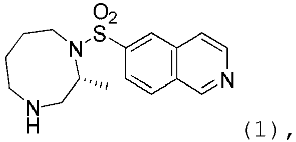 an acid addition salt thereof, or a solvate of the compound or the acid addition salt.
[8] The method according to [7], wherein the administration means is eye-drop administration.

### Advantageous Effects of Invention

According to the present invention, it is possible to provide a pharmaceutical composition for preventing and/or treating ocular complications in cGVHD.

### Brief Description of Drawings

Figure 1 illustrates the GVHD scores of a 0.5% compound 1 (H-1129) group, a vehicle group, and a control group.
Figure 2 illustrates the tear volumes measured by the cotton thread method in the 0.5% compound 1 (H-1129) group, the vehicle group, and the control group.
Figure 3 illustrates effects on major transcription factor Snail-positive cells indicating epithelial-mesenchymal transition in the 0.5% compound 1 (H-1129) group, the vehicle group, and the control group.
Figure 4 illustrates effects on eyelid fibrosis in the 0.5% compound 1 (H-1129) group, the vehicle group, and the control group.

### Description of Embodiments

The terms used in the present specification are used in the sense commonly used in the art, unless otherwise specified.

The term "contain" or "comprise" in the present specification includes all cases of "consisting of," "consisting essentially of," and "optionally further containing other components."

Further, the phrase "comprising . . . as an active ingredient" means that the applicable ingredient is contained as an active ingredient, and this phrase is not intended to prevent containing other ingredients.

In addition, the phrase "prevention/treatment" includes all of prevention, treatment, and prevention and treatment.

Graft-versus-host disease (GVHD) in the present specification is one of the complications associated with hematopoietic stem cell transplantation, such as organ transplantation and bone marrow transplantation, and is a general term for symptoms which occur when the organs of the donor (organ source) attack the organs of the recipient through an immune response. GVHD includes acute GVHD, which occurs about one to two weeks after transplantation, and chronic GVHD, which occurs regardless of the time of onset after transplantation, including conventional chronic GVHD (cGVHD) and overlap syndrome, which is a mixture of cGVHD and acute GVHD. The target of the present invention is preferably cGVHD.

cGVHD occurs in various tissues and organs, and ocular complications in cGVHD include dry eyes, meibomian gland dysfunction, retinal hemorrhage, acute conjunctivitis, and the like. In the present invention, all of these ocular complications are targeted. Of these ocular complications, dry eyes are most frequent; thus, it is particularly preferable to target dry eyes.

An aspect of the present invention is a pharmaceutical composition for preventing/treating an ocular complication in cGVHD, the composition comprising (R)-6-(2-methyl-1,4-diazocan-1-ylsulfonyl)isoquinoline of the above formula (1) (hereinafter also referred to as "compound 1"), an acid addition salt thereof, or a solvate of the compound or the acid addition salt, as an active ingredient.

The compound 1, which is the active ingredient of the pharmaceutical of the present invention, is described in Patent Literature 1, and is known to be effective for diseases or disorders caused by glaucoma, cardiovascular disease, or neurodegeneration or nerve damage; however, its preventive or therapeutic effects for ocular complications in cGVHD are not known.

The compound 1 can be produced by the method described in Patent Literature 1, and can also be used in the form of an acid addition salt or a solvate of the compound or the acid addition salt. Examples of acids which form acid addition salts include salts of inorganic acids, such as hydrochloric acid, sulfuric acid, nitric acid, phosphoric acid, hydrofluoric acid, and hydrobromic acid; and salts of organic acids, such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid, and camphorsulfonic acid. Examples of solvates include hydrates, ethanolates, and the like.

The compound 1 has the effect of reducing the symptoms of ocular complications in cGVHD, such as increased tear secretion and fibrosis of eyelid tissue, as shown in the Examples provided later, is effective as a drug for preventing/treating ocular complications in cGVHD, and is particularly useful as a drug for preventing/treating dry eyes in cGVHD. The compound 1 is also useful for the prevention/treatment of meibomian gland dysfunction in cGVHD due to its effect of reducing fibrosis of eyelid tissue by inhibiting epithelial-mesenchymal transition.

The pharmaceutical composition of the present invention comprises the compound 1, an acid addition salt thereof, or a solvate of the compound 1 or the acid addition salt, as an active ingredient, and may contain a pharmaceutically acceptable carrier suitable for the form (dosage form) of the composition.

The route of administration of the pharmaceutical composition of the present invention may be oral or parenteral. In the case of parenteral administration, eye drops, eye ointments, or subconjunctival injections are preferred. Examples of the dosage form include tablets, capsules, granules, powders, injections, eye drops, eye ointments, and the like, with eye drops, eye ointments, or injections being preferred.

For example, oral agents, such as tablets, capsules, granules, and powders, can be prepared by optionally combining the compound 1 with excipients, such as lactose, mannitol, starch, crystalline cellulose, light anhydrous silicic acid, calcium carbonate, and calcium hydrogen phosphate; lubricants, such as stearic acid, magnesium stearate, and talc; binders, such as starch, hydroxypropylcellulose, hydroxypropylmethylcellulose, and polyvinylpyrrolidone; disintegrants, such as carboxymethylcellulose, low-substituted hydroxypropylmethylcellulose, and calcium citrate; coating agents, such as hydroxypropylmethylcellulose, macrogol, and silicone resin; stabilizers, such as ethyl parahydroxybenzoate and benzyl alcohol; and flavoring agents, such as sweeteners, acidulants, and flavorings.

Parenteral agents, such as eye drops and injections, can be prepared, for example, by optionally combining the compound 1 with isotonic agents, such as glycerin, propylene glycol, sodium chloride, potassium chloride, sorbitol, and mannitol; buffers, such as phosphoric acid, phosphate, citric acid, glacial acetic acid, ε-aminocaproic acid, and trometamol; pH adjusters, such as hydrochloric acid, citric acid, phosphoric acid, glacial acetic acid, sodium hydroxide, potassium hydroxide, sodium carbonate, and sodium bicarbonate; solubilizing or dispersing agents, such as polysorbate 80, polyoxyethylene hydrogenated castor oil 60, macrogol 4000, refined soy lecithin, and polyoxyethylene (160) polyoxypropylene (30) glycol; cellulose polymers, such as hydroxypropylmethylcellulose and hydroxypropylcellulose; thickeners, such as polyvinyl alcohol and polyvinylpyrrolidone; stabilizers, such as edetic acid and sodium edetate; preservatives or antiseptics, such as general-purpose sorbic acid, potassium sorbate, benzalkonium chloride, benzethonium chloride, methyl parahydroxybenzoate, propyl parahydroxybenzoate, and chlorobutanol; and soothing agents, such as chlorobutanol, benzyl alcohol, and lidocaine. The pH is preferably set to from 4.0 to 8.0, and the osmotic pressure ratio is preferably set to around 1.0. Eye ointments can be formulated by optionally using commonly used bases, such as white petrolatum and liquid paraffin.

The dosage can be appropriately selected and used depending on the symptom, age, dosage form, and the like. For example, an oral agent can be generally administered at a daily dose of from 0.01 to 1000 mg, and preferably from 1 to 100 mg, once a day or in divided doses.

In the case of an eye drop, one with a concentration of from 0.0001% to 10% (w/v), and preferably from 0.01% to 5% (w/v), can be generally administered once a day or in divided doses. In the case of an injection, one with a concentration of from 0.0001 mg to 1000 mg/mL can be generally administered once a day or in divided doses. In the case of an eye ointment, one with a concentration of from 0.01% to 10% (w/w), and preferably from 0.1% to 5% (w/w), can be generally administered once a day or in divided doses.

### Examples

Now, the present invention will be described in more detail with reference to Examples; however, the present invention is not limited thereto.

### (Test Example 1)

### <Evaluation of ROCK inhibitory effect>

Compounds 1 with various concentrations were used to calculate the concentration which inhibits 50% of the phosphorylation reaction of substrates by ROCK1 or ROCK2 in vitro (hereinafter referred to as "IC₅₀ value"). The results are shown in Table 1.

**[Table 1]**

| | ROCK1 inhibitory effect/ IC50 value | ROCK2 inhibitory effect/ IC50 value |
|---|---|---|
| Compound 1 | 0.52 µM | 0.23 µM |

### <Evaluation of effects of improving ocular complications using cGVHD model animals>

cGVHD model mice were used to evaluate whether drug administration improved ocular complications in cGVHD.

The mice were created in the following manner.

Bone marrow transplantation was performed between 7-and 9-week-old allogeneic mice. Specifically, the transplantation was performed using male B10.D2 mice as donors and female BALB/c mice as recipients. This transplantation is hematopoietic stem cell transplantation with matched major histocompatibility complex (MHC) and mismatched minor histocompatibility antigen (MiHA). As controls, non-cGVHD models were also created, in which syngeneic hematopoietic stem cell transplantation was performed (donors and recipients were both BALB/c mice). The transplant method was as follows. The recipient was irradiated with 7 Gy of radiation, then 1 × 10⁶ bone marrow cells and 2 × 10⁶ spleen cells were each dissolved in 0.1 mL of RPMI solution and placed in one syringe, and a total of 0.2 mL was administered via the tail vein. The transplantation was carried out in a specific pathogen free (SPF) environment, and all experimental animals were similarly fed sterile water and food. The cGVHD model mice and non-cGVHD model mice were each analyzed 4 weeks after transplantation.

The mice were divided into a treatment group in which the compound 1 (concentration: from 0.02% to 0.5%) was administered by eye drops to GVHD model mice (hereinafter referred to as the H-1129 group), and a vehicle group in which phosphate-buffered saline (PBS) as a placebo was administered by eye drops to GVHD model mice. These two groups were compared with a control group in which PBS as a placebo was administered by eye drops to non-GVHD mice.

The eye drops were administered from the day before transplantation until the 27th day after transplantation. On the last day, the systemic GVHD scores and the tear volume by the cotton thread method were measured. The mice were dissected 28 days after transplantation, and the eyelids, eyeballs, and lacrimal glands were histopathologically examined.

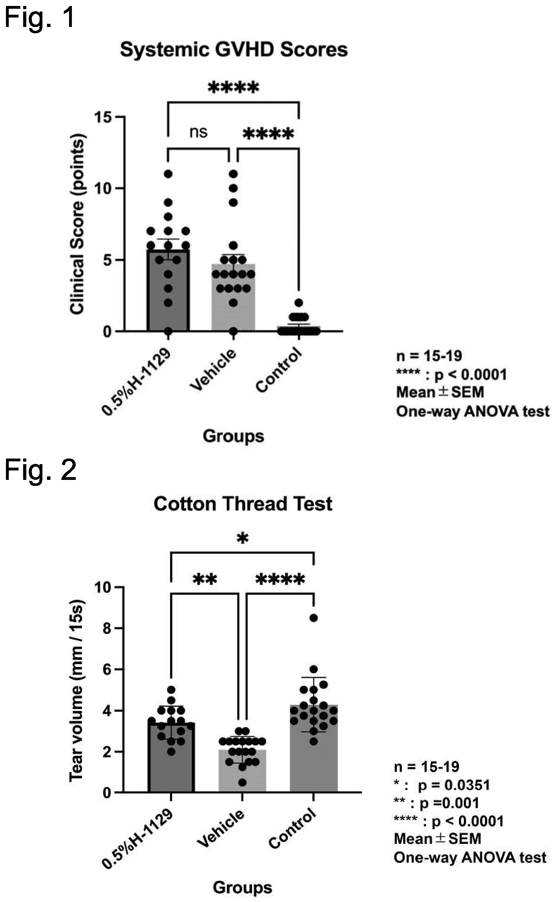

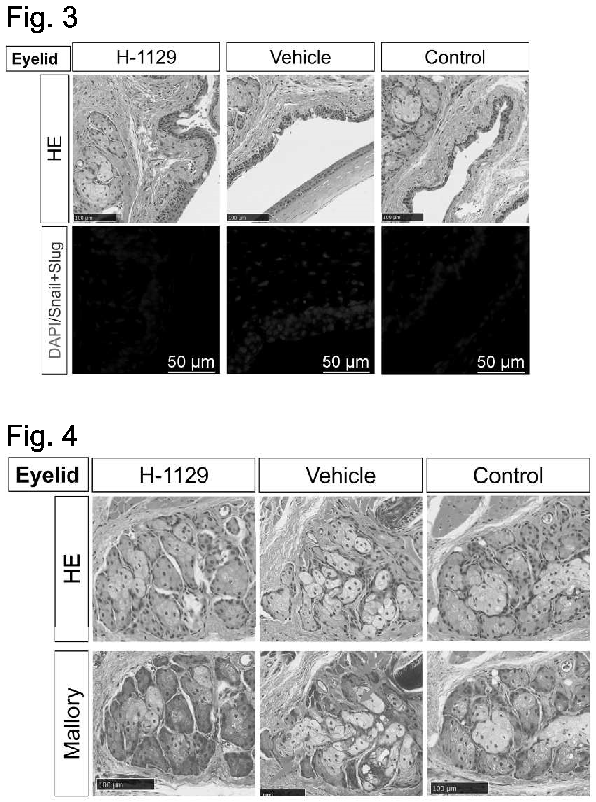

The 0.5% compound 1 (indicated as H-1129 in the figure) group and the vehicle group showed significantly higher GVHD scores in comparison to the control group, showing the onset of chronic GVHD (Figure 1). The amount of tear secretion was significantly improved in the 0.5% compound 1 (indicated as H-1129 in the figure) group (3.42 ± 0.41 mm) in comparison to the vehicle group (2.09 ± 0.29 mm) (p<0.001), and there was no significant difference from the control group (4.29 ± 0.59 mm) (Figure 2). In comparison to the vehicle group, eyelid fibrosis was improved in the 0.5% compound 1 (indicated as H-1129 in the figure) group, and there were few major transcription factor Snail-positive cells indicating epithelial-mesenchymal transition (Figures 3 and 4).

It is evident from the above results that the compound 1 has ROCK inhibitory effects, and improves symptoms of ocular complications in cGVHD, particularly dry eyes. In addition, the compound 1 inhibits epithelial-mesenchymal transition, which reduces fibrosis of eyelid tissue. This action is a mechanism of symptomatic improvement of dry eyes, and also suggests that the compound 1 may be useful in the prevention/treatment of other ocular complications, such as meibomian gland dysfunction.

### (Preparation Example 1)

### Eye drop (in 100 mL)

| | |
|---|---|
| Compound 1 | 0.5 g |
| Sodium dihydrogen phosphate hydrate | 0.6 g |
| Disodium hydrogen phosphate hydrate | 1.4 g |
| Benzalkonium chloride | 0.01 g |
| Sodium chloride | 0.3 g |
| Dilute hydrochloric acid | appropriate amount |
| Sodium hydroxide | appropriate amount |
| Purified water | appropriate amount |

### (Preparation Example 2)

### Eye drop (in 100 mL)

| | |
|---|---|
| Compound 1 | 0.5 g |
| Boric acid | 0.2 g |
| Concentrated glycerin | 0.25 g |
| Benzalkonium chloride | 0.005 g |
| Dilute hydrochloric acid | appropriate amount |
| Sodium hydroxide | appropriate amount |
| Purified water | appropriate amount |

### (Preparation Example 3)

### Eye drop (in 100 mL)

| | |
|---|---|
| Compound 1 | 0.5 g |
| Sodium edetate | 0.05 g |
| Concentrated glycerin | 2.0 g |
| Benzalkonium chloride | 0.005 g |
| Dilute hydrochloric acid | appropriate amount |
| Sodium hydroxide | appropriate amount |
| Purified water | appropriate amount |

### (Preparation Example 4)

### Eye drop (in 100 mL)

| | |
|---|---|
| Compound 1 | 0.5 g |
| Sodium dihydrogen phosphate | 0.8 g |
| Sodium chloride | 0.5 g |
| Benzalkonium chloride | 0.005 g |
| Sodium hydroxide | appropriate amount |
| Purified water | appropriate amount |

## Claims

1. A pharmaceutical composition for preventing/treating an ocular complication in cGVHD, the composition comprising (R)-6-(2-methyl-1,4-diazocan-1-ylsulfonyl)isoquinoline of the following formula (1): 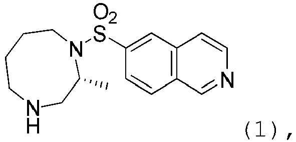 an acid addition salt thereof, or a solvate of the compound or the acid addition salt, as an active ingredient.

2. The pharmaceutical composition according to claim 1, wherein the composition is in a form of an eye drop composition.

3. (R)-6-(2-methyl-1,4-diazocan-1-ylsulfonyl)isoquinoline of the following formula (1): 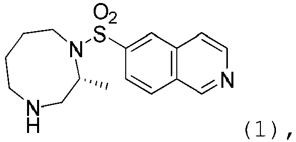 an acid addition salt thereof, or a solvate of the compound or the acid addition salt, for preventing/treating an ocular complication in cGVHD.

4. The compound according to claim 3, wherein the pharmaceutical is in a form of an eye drop.

5. Use of (R)-6-(2-methyl-1,4-diazocan-1-ylsulfonyl)isoquinoline of the following formula (1): 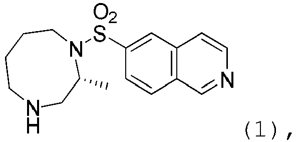 an acid addition salt thereof, or a solvate of the compound or the acid addition salt, for producing a drug for preventing/treating an ocular complication in cGVHD.

6. The use according to claim 5, wherein the pharmaceutical is in a form of an eye drop.

7. A method for preventing/treating an ocular complication in cGVHD, the method comprising administering (R)-6-(2-methyl-1,4-diazocan-1-ylsulfonyl)isoquinoline of the following formula (1): 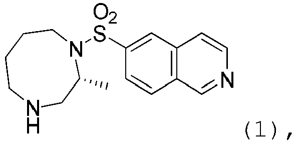 an acid addition salt thereof, or a solvate of the compound or the acid addition salt.

8. The method according to claim 7, wherein the administration means is eye-drop administration.
